# EUROPÄISCHE PATENTSCHRIFT

(11) **EP 0 407 820 B1**
(45) Veröffentlichungstag und Bekanntmachung des Hinweises auf die Patenterteilung: **09.03.1994**
(21) Anmeldenummer: 90112328.1
(22) Anmeldetag: 28.06.1990
(51) Int. Cl.: A61M 13/00, A61M 1/00, A61B 1/12

(54) **Gerät zur Insufflation und Reinigung von Gas**
Gas insufflation and cleaning apparatus
Appareil d'insufflation et de filtration d'un gaz

(30) Priorität: 11.07.1989 DE 3922746
(43) Veröffentlichungstag der Anmeldung: 16.01.1991
(73) Patentinhaber: Richard Wolf GmbH, 75438 Knittlingen (DE)
(72) Erfinder: Baier, Manfred, D-7134 Knittlingen (DE)
(74) Vertreter: Wilcken, Thomas, Dipl.-Ing.

(56) Entgegenhaltungen:
- EP-A- 0 169 972
- EP-A- 0 293 669
- EP-A- 0 316 593
- DE-A- 3 000 218
- DE-A- 3 601 118
- DE-C- 3 611 018
- FR-A- 2 326 734
- US-A- 4 735 603

## Beschreibung

Die Erfindung betrifft ein Insufflationsgerät für die Anwendung bei endoskopischen Eingriffen, insbesondere für die Humanmedizin gemäß dem Oberbegriff des Patentanspruchs 1.

Solche Geräte werden benötigt, um eine im Wege eines endoskopischen Eingriffes zu untersuchende und zu behandelnde Körperhöhle in dafür notwendiger Weise aufzuweiten. Dabei ist es erforderlich, den Innendruck in der Körperhöhle während des Eingriffes möglichst konstant zu halten.

Aus der DE-A-37 39 003 ist ein Insufflationsgerät bekannt, welches mit zwei Strömungswegen ausgestattet ist, und welches in der Lage ist, geringe Gasverluste stetig auszugleichen. Größere Gasverluste, die durch Instrumentenwechsel oder dergleichen verursacht sein können, werden durch Umschalten auf den zweiten Strömungsweg innerhalb der vorgewählten Druckgrenze schnell ausgeglichen. Außerdem ist gewährleistet, daß bei Überschreiten des vorgewählten Druckgrenzwertes die Zuführungsleitung und eine Pumpe sowie weitere an den Ausgängen angeschlossene und von einer Auswerteelektronik angesteuerte Zusatzgeräte außer Betrieb gehen und keine weitere Zufuhr von Gas in die Körperhöhle erfolgt.

Die DE-C-33 29 784 zeigt weiter ein solches Gerät, das für den Einsatz von Koagulationgsinstrumenten, wie z. B. für Laseranwendung geeignet ist. Dabei besteht das Erfordernis, den bei der Koagulation entstehenden Rauch möglichst schnell abzuziehen, da dieser die visuelle Kontrolle des Eingriffes beeinträchtigt, wobei dieser Vorgang durchgeführt werden muß, ohne daß sich die Druckverhältnisse in der Körperhöhle ändern. Dies geschieht bei diesem Stand der Technik, indem der Rauch zusammen mit Sekret und Insufflationsgas abgesaugt wird, wobei das Gas durch einen Filter gereinigt und über einen geschlossenen Kreislauf in die Körperhöhle zurückgeführt wird.

In der US-A-4 735 603 ist eine für die Laparaskopie bestimmte Einrichtung gezeigt, die einerseits aus einem gesonderten Insufflationsgerät, mit dem eine große Körperhöhle eines Patienten aufgeweitet wird, und andererseits aus einem von dem Insufflationsgerät getrennten, geschlossenen Kreislaufsystem besteht, mit dem verunreinigtes Gas aus der Körperhöhle des Patienten abgesaugt wird, um es u.a. von dem durch die Laserbehandlung entstandenen Rauch zu befreien und dann wieder in die Körperhöhle zurückzuleiten. Das Kreislaufsystem umfaßt neben einer Zirkulationgsleitung eine Umwälzpumpe, Filter für Rauch und Bakterien und eine elektrische Steuereinheit zur Steuerung von Ventilen, um beim ständigen Austausch des CO₂-Gases in der Körperhöhle dessen Druck in den zulässigen Grenzen einzuhalten. Nachdem die Körperhöhle durch direkte Einleitung von CO₂-Gas vom gesonderten Insufflationsgerät aufgeweitet worden ist, wird das Kreislaufsystem eingeschaltet und die Laserbehandlungstätigkeit begonnen, um einen ständigen Gasaustausch bei vorbestimmtem Druck vornehmen zu können.

Die bekannten Insufflationsgeräte einschließlich der erwähnten Einrichtung, die zum gewünschten schnellen Druckausgleich hohe Gasflußraten aufweisen, sind für eine Endoskopie kleiner Körperhöhlen, wie z.B. dem Uterus, nicht geeignet, da es hier außerdem darauf ankommt, daß die mit den physiologischen Gegebenheiten vertretbare maximale Gaszufuhr nicht überschritten wird.

Die Aufgabe der vorliegenden Erfindung besteht darin, den Nachteil, daß die vorgenannten Insufflationseinrichtungen für endoskopische Eingriffe in kleinen Körperhöhlen nicht geeignet sind, zu vermeiden.

Die Lösung dieser Aufgabe geht von dem einleitend angeführten Insufflationsgerät aus und kennzeichnet sich weiter durch die Merkmale im Kennzeichenteil des Patentanspruchs 1.

Die mit der Erfindung erzielbaren Vorteile bestehen darin, daß das Insufflationsgerät für die gezielte Ausdehnung von kleinen Körperhöhlen geeignet ist und neben der Konstanthaltung der Druckverhältnisse in der so innerhalb der kleinen Körperhöhle geschaffenen Gasblase in der Lage ist, Störungen im Absaugkreis zu erkennen und darauf so zu reagieren, daß keine den Patienten gefährdenden Druckverhältnisse auftreten können. Darüber hinaus ist das erfindungsgemäße Insufflationsgerät imstande, eine Insufflation zu blockieren, wenn das für einen Eingriff angeschlossene Gerät hinsichtlich seiner Gas-Durchsatzleistung ungeeignet ist, d.h. ein Gerät mit höheren möglichen Flußraten angeschlossen ist.

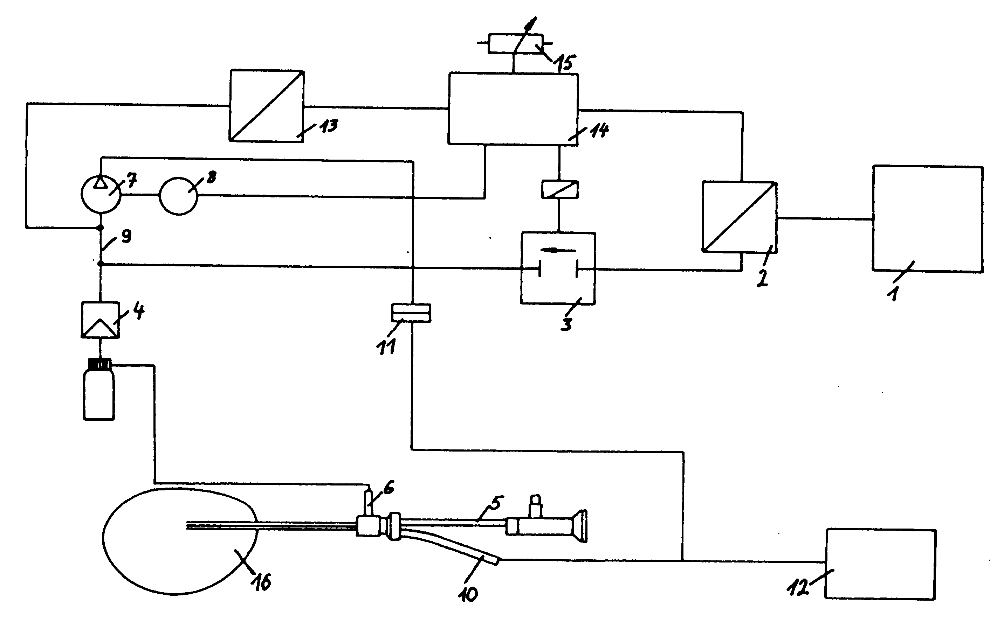

Die Erfindung ist nachfolgend anhand eines in der Zeichnung dargestellten Ausführungsbeispieles näher erläutert. Die einzige Figur zeigt das Blockschaltbild eines nach der Erfindung arbeitenden Insufflationsgerätes.

Das Blockschaltbild weist einen Gas-Steuerkreis auf, der ein geeignetes Gasversorgungsgerät 1 über einen Durchflußmesser 2, ein Absperrventil 3 und ein Filter 4 über einen Anschlußstutzen 6 mit einem sich anschließenden Instrumentenkanal des Endoskopes 5 verbindet. Das Blockschaltbild zeigt weiter einen Absaugkreis mit einer durch einen Motor 8 angetriebenen Pumpe 7, deren Saugseite 9 mit dem Ausgang des Absperrventils 3 und mit dem Filter 4 verbunden ist und welche über ein Sterilfilter 11 an einen weiteren Instrumentenkanal 10 des Endoskopes 5 angeschlossen ist, und zwar über die den Lichtleiter in einem Abstand umgebende Umhüllung des Laserlichtleiters eines Lasergerätes 12. Die Druckverhältnisse an der Saugseite 9 der Pumpe 7 werden durch einen Druck-Meßwandler 13 registriert, der, wie auch der Durchflußmesser 2, die Meßdaten an einen eine Auswerteelektronik 14 gibt, die die Meßdaten mit dem durch den Sollwertgeber 15 voreingestellten Wert vergleicht, wobei die Ausgänge der Auswerteelektronik 14 mit dem Absperrventil 3 und dem Motor 8 der Pumpe 7 verbunden sind.

Der Durchflußmesser 2 überwacht den Grenzwert des Flow und steuert über die Auswerteelektronik 14 das Absperrventil 3. Dazu wird in der Auswerteelektronik 14 ein Soll-Ist-Wert-Vergleich durchgeführt und bei Überschreiten des zulässigen Flow das Absperrventil 3 geschlossen. Zur Ausdehnung einer Körperhöhle 16 erfolgt ein Gasfluß über das Absperrventil 3. Das Gas gelangt bei noch nicht arbeitender Pumpe 7 über das Filter 4 in die Körperhöhle 16. Befindet sich bei Inbetriebnahme eine Leckstelle im Gaskreislauf, so wird, bedingt durch die geringe Flußleistung der Gasversorgungseinheit, der eingestellte Drucksollwert nicht erreicht und die Pumpe kann nicht eingeschaltet werden.Bei Erreichen des über den Sollwertgeber 15 vorgewählten Druckes wird das Absperrventil 3 betätigt und damit der Gaszufluß aus dem Gasversorgungsgerät 1 gesperrt.Wird nun mit dem Lasergerät 12 gearbeitet,so kann zum Absaugen des entstehenden Rauches die Pumpe 7 eingeschaltet werden. Dabei ist die Pumpe nur einschaltbar, wenn der vorgewählte Druck erreicht ist. Der Rauch wird so mit dem Gas aus der Körperhöhle 16 abgesaugt, in dem Filter 4 ausgefiltert und das gereinigte Gas über das zur Verhinderung der Keimverschleppung eingebaute Sterilfilter 11 in die Körperhöhle 16 zurückgepumpt.

Der Soll-Druckwert kann zwischen 40 mbar und 200 mbar vorgegeben werden. Bei Unterschreiten des vorgewählten Druckes um einen Betrag von ca. 20 mbar wird so lange kein Gas umgewälzt, bis durch das Gasversorgungsgerät 1 der Druck wieder auf den Sollwert gebracht wurde. Die Druckmessung erfolgt durch den Druck-Meßwandler 13 an der Saugseite 9 der Pumpe 7, da hier über das Filter 4 und den großen Durchflußquerschnitt des Endoskopes 5 beim angestrebten Filterdurchlaß von 500 bis 1000 ml/min keine wesentliche Druckdifferenz zur Körperhöhle besteht. Es wird somit ein geschlossener Gaskreislauf sichergestellt, da bei Gasverlusten im Kreislauf die Änderung des vorgewählten Druckwertes durch den Druck-Meßwandler 13 erfaßt wird. Bei Messung des Druckes auf der Druckseite der Pumpe 7 würden die Leitungen von in der Regel engem Querschnitt (Lichtleiterummantelung) eine Druckverfälschung hervorrufen. Leckstellen in den Verbindungsleitungen oder dem Endoskop 5 bedingen einen Druckabfall in dem Absaugkreis, der von der Auswerteelektronik 14 erkannt wird und zur Abschaltung der Pumpe 7 führt. Der Verschluß einer Leitung oder das Knicken eines Schlauches lassen den Druck unter den Sollwert abfallen und werden als Störung des Kreislaufes erkannt. Bei Fehlen einer Gasversorgung ist das Ansaugen von Luft ausgeschlossen, da die Pumpe 7 abgeschaltet ist.

Durchflußmesser 2, Auswerteelektronik 14 und Absperrventil 3 können auch so ausgelegt sein, daß bei Benutzung eines CO₂-Versorgungsgerätes mit höherem Flow dieser auf den für die Anwendung zulässigen Höchstwert begrenzt wird. Sind am Endoskop 5 genügend Anschlüsse vorhanden, so muß die Lichtleiterummantelung nicht zur Zuführung des Gases in dem Gaskreislauf integriert sein. Dieser kann dann über weitere Anschlüsse hergestellt und der Lichtleiter über ein geeignetes Zwischenstück in einen separaten Instrumentenkanal eingeführt werden.

## Patentansprüche

1. Insufflationsgerät für die Anwendung bei endoskopischen Eingriffen, mit dem ein aus einem Gasversorgungsgerät (1) über einen Instrumentenkanal des Endoskopes (5) in eine Körperhöhle, beispielsweise den Uterus, einleitbares Gas hinsichtlich Druck und Durchflußmenge überwacht wird und über einen Absaugkeis mit Filter (4) zum Ausfiltern von Rauch und/oder Dampf führbar ist, wobei der Absaugkreis eine mit ihrer Saugseite (9) zwischen dem Filter (4) und dem Absperrventil (3) angeschlossene, mit einem weiteren Instrumentenkanal (10) des Endoskopes (5) verbundene Pumpe (7) und einen an der Saugseite (9) der Pumpe (7) angeschlossenen Druckmeßwandler (13) sowie ein dieser Pumpe nachgeordnetes Sterilfilter (11) umfaßt dadurch gekennzeichnet, daß das Gas auf seinem Wege zu dem genannten Instrumentenkanal des Endoskopes (5) einen Durchflußmesser (2), ein Absperrventil (3) und das Filter (4) durchströmt und daß die Überwachung durch eine Auswerteelektronik (14) erfolgt, die ihre Steuerdaten aus dem Druck-Meßwandler (13) sowie aus dem Durchflußmesser (2) bezieht und das Absperrventil (3) und die Pumpe (7) durch Vergleich des Ist-Druckwertes mit einem vorgegebenen Soll-Druckwert steuert.

## Claims

1. An insufflation apparatus for application in endoscopic operations with which a gas, which is able to be introduced from a gas supply apparatus (1) via an instrument channel of the endoscope (5) into a body cavity, for example the uterus, is monitored as regards pressure and amount of flow and is able to be carried via a suction circuit with filter (4) for the filtering out of fumes and/or steam, in which the suction circuit comprises a pump (7) connected with its suction side (9) between the filter (4) and the shut-off valve (3), connected with a further instrument channel (10) of the endoscope (5), and a pressure measuring transformer (13), connected to the suction side (9) of the pump (9), and also a sterile filter (11) arranged after this pump, characterised in that the gas, on its way to said instrument channel of the endoscope (5) flows through a flow meter (2), a shut-off valve (3) and the filter (4), and that the monitoring takes place through an evaluation electronic arrangement (14), which draws its control data from the pressure measuring transformer (13) and also from the flow meter (2) and controls the shut-off valve (3) and the pump (7) through comparison of the actual pressure value with a predetermined nominal pressure value.

## Revendications

1. Appareil d'insufflation pour application dans des interventions endoscopiques, permettant de contrôler, en termes de pression et de débit, un gaz susceptible d'être acheminé à partir d'un appareil d'alimentation en gaz (1) via un canal instrumental de l'endoscope (5) dans une cavité corporelle, par exemple, l'utérus, et de l'acheminer à travers un circuit d'aspiration pourvu d'un filtre (4) pour filtrer la fumée et/ou la vapeur, le circuit d'aspiration comprenant une pompe (7) raccordée par son côté d'aspiration (9) entre le filtre (4) et la soupape d'arrêt (3), reliée à un autre canal instrumental (10) de l'endoscope (5), et un transducteur de pression (13) raccordé au côté d'aspiration (9) de la pompe (7), ainsi qu'un filtre stérile (11) agencé en aval de cette pompe, caractérisé en ce que le gaz s'écoule, sur son chemin vers ledit canal instrumental de l'endoscope (5) à travers un débitmètre (2), une soupape d'arrêt et le filtre (4), et la surveillance se fait par un circuit électronique d'évaluation (14) qui tire ses données de commande du transducteur de pression (13) ainsi que du débitmètre (2) et qui commande la soupape d'arrêt (3) de la pompe (7) par comparaison de la valeur de la pression réelle avec une valeur de la pression de consigne prédéterminée.
